Europäisches Patentamt

European Patent Office

Office européen des brevets

(19)

(11) Numéro de publication : **0 688 834 A1**

(12)

# DEMANDE DE BREVET EUROPEEN

(21) Numéro de dépôt : **95401442.9**

(22) Date de dépôt : **19.06.95**

(51) Int. Cl.⁶ : **C09C 3/12,** C09C 1/36,
C09C 1/30, C08K 9/06,
A61K 7/42, C09D 17/00

(30) Priorité : **24.06.94 FR 9407832**

(43) Date de publication de la demande :
**27.12.95 Bulletin 95/52**

(84) Etats contractants désignés :
**AT BE CH DE DK ES FR GB GR IE IT LI LU MC
NL PT SE**

(71) Demandeur : **RHONE-POULENC CHIMIE
25, Quai Paul Doumer
F-92408 Courbevoie Cédex (FR)**

(72) Inventeur : **Labarre, Dominique
18 bis, rue de Chartres
F-92200 Neuilly sur Seine (FR)**

(74) Mandataire : **Polus, Camille et al
c/o Cabinet Lavoix
2, Place d'Estienne d'Orves
F-75441 Paris Cedex 09 (FR)**

(54) **Procédé de préparation de particules d'oxyde métallique organophiles**

(57) On prépare des particules organophiles d'un oxyde métallique choisi parmi l'alumine $Al_2O_3$, la silice $SiO_2$, les oxydes de métaux de transition, notamment l'oxyde de titane $TiO_2$, et les oxydes de terres rares, notamment l'oxyde de cérium $CeO_2$, en faisant réagir une suspension hydroalcoolique (a) de particules d'oxyde métallique dont la surface ne possède pas de pores de diamètre inférieur à 5 nm, avec une solution alcoolique anhydre (b) comprenant au moins un alcoxysilane de formule (1)
$$Si(OR)_x R'_{4-x} \quad (1)$$
dans laquelle R représente un groupe alkyle comportant de 1 à 6 atomes de carbone, R' représente un groupe hydrocarboné choisi parmi les groupes alkyle, cycloalkyle, aryle, alkylaryle, arylalkyle, alcényle et alcynyle comportant au moins 1 atome de carbone et x est un entier de 1 à 3, lesdits alcools, identiques ou différents, comportant de 1 à 5 atomes de carbone.

EP 0 688 834 A1

La présente invention concerne un procédé de préparation de particules d'oxyde métallique organophiles.

L'utilisation de particules minérales comme charge, pigment ou additif est courante dans des domaines aussi divers que les peintures, les plastiques ou les cosmétiques. Des problèmes se posent lorsque ces particules minérales hydrophiles doivent être incorporées dans des matrices organiques solides ou liquides, car leur faible affinité pour de tels milieux se traduit par une mauvaise dispersion dans la matrice.

Or, l'efficacité du pigment ou de la charge est optimale lorsque l'état de dispersion des particules est maximal. On s'emploie donc à obtenir des suspensions de particules aussi dispersées que possible.

Pour être dispersible, la particule doit être "mouillable" par la matrice, c'est-à-dire que sa surface doit être rendue compatible avec la matrice organique.

En pratique, on introduit dans la suspension organique un agent dit "compatibilisant" qui interagit avec les particules pour rendre la suspension stable. Les particules ainsi incorporées dans la matrice organique sont souvent agglomérées et on améliore l'état de dispersion en désagglomérant la suspension en y ajoutant un agent de stabilisation stérique des particules. En formant un enrobage de quelques nanomètres autour de la particule minérale compatibilisée, une telle molécule facilite la dispersion de particules désagglomérées.

Ainsi, les dispersions de particules minérales en milieu organique sont classiquement obtenues par malaxage/broyage du support minéral dans un solvant organique en présence d'une molécule tensioactive qui joue à la fois le rôle d'agent mouillant et de stabilisateur stérique (la chaîne carbonée traînante de la molécule tensioactive suffisant à former un enrobage de la particule). Cependant, lorsque les particules minérales sont de taille nanométrique (c'est-à-dire lorsqu'elles ont un diamètre inférieur à 0,1 μm), ce mode de traitement permet difficilement d'atteindre un état de dispersion maximale.

En outre, l'interaction entre la surface du minéral et la molécule compatibilisante, qui peut être par exemple un acide gras, un alcool gras ou un tensioactif anionique, présente un caractère de réversibilité relativement prononcé qui entraîne une certaine instabilité de la dispersion.

On préfère établir une interaction irréversible entre l'agent compatibilisant et la particule minérale en formant une liaison covalente entre un site réactif de la particule et une fonction réactive de la molécule compatibilisante. La particule minérale ainsi modifiée est rendue organophile de façon irréversible et peut être isolée, et utilisée aisément ultérieurement pour toute application en milieu organique.

La présente invention s'inscrit dans ce cadre, puisqu'elle a pour but de fournir un procédé de préparation de particules minérales organophiles dispersibles dans un solvant organique.

En effet, les présents inventeurs ont découvert que lorsque l'on effectue la compatibilisation de particules d'oxyde métallique hydrophile par greffage en surface de groupements alkylsilane en milieu hydroalcoolique, on peut aboutir à des particules finales hautement dispersibles en milieu organique si l'on travaille avec des particules d'oxyde de départ "non-nanoporeuses", c'est-à-dire ne présentant pas à leur surface de pores de diamètre inférieur à 5 nm.

La présente invention a ainsi pour objet un procédé de préparation de particules organophiles d'un oxyde métallique choisi parmi l'alumine $Al_2O_3$, la silice $SiO_2$, les oxydes des métaux de transition, notamment l'oxyde de titane $TiO_2$, et les oxydes de terres rares, notamment l'oxyde de cérium $CeO_2$, par lequel on fait réagir une suspension hydroalcoolique (a) de particules d'oxyde métallique dont la surface ne possède pas de pores de diamètre inférieur à 5 nm avec une solution alcoolique anhydre (b) comprenant au moins un alcoxysilane de formule (1)

$$Si(OR)_x R'_{4-x} \qquad (1)$$

dans laquelle R représente un groupe alkyle comportant de 1 à 6 atomes de carbone, R' représente un groupe hydrocarboné choisi parmi les groupes alkyle, cycloalkyle, aryle, alkylaryle, arylalkyle, alcényle et alcynyle comportant au moins 1 atome de carbone et x est un entier de 1 à 3, lesdits alcools, identiques ou différents, comportant de 1 à 5 atomes de carbone.

Ce procédé peut s'appliquer en particulier à des "nanoparticules" d'oxyde métallique, c'est-à-dire à des particules ayant un diamètre moyen inférieur ou égal à 100 nm. Les procédés classiques de compatibilisation employés jusqu'à présent ne permettent pas de conférer à ces particules de petite taille les bonnes propriétés de dispersibilité obtenues par le procédé selon l'invention, et qui seront explicitées par la suite.

Dans le cas où les particules d'oxyde à traiter sont "nanoporeuses", c'est-à-dire qu'elles présentent en surface des pores de diamètre inférieur à 5 nm, le procédé comporte une étape préliminaire qui consiste à éliminer cette nanoporosité.

Cette étape préliminaire peut comprendre un traitement thermique des particules nanoporeuses en autoclave. Un tel traitement est décrit notamment dans la demande de brevet FR-A-2 677 012 déposée par la demanderesse.

On peut également effectuer un traitement chimique des particules nanoporeuses. Les particules d'oxyde sont alors mises en suspension dans un milieu hydroalcoolique, l'alcool comportant de 1 à 5 atomes de carbone, et on fait réagir la suspension (c) ainsi obtenue avec au moins un tétraalcoxysilane de formule (2)

EP 0 688 834 A1

$$Si(OR'')_4 \qquad (2)$$

dans laquelle R'' représente un groupe alkyle comportant de 1 à 5 atomes de carbone.

Le tétraalcoxysilane est ajouté peu à peu à la suspension. En fin de traitement, on obtient une suspension hydroalcoolique (a) stable de particules minérales non nanoporeuses, suspension (a) que l'on peut alors traiter directement avec un alcoxysilane de formule (1) comme indiqué précédemment pour la compatibilisation proprement dite.

Dans le cas où l'on dispose de particules non nanoporeuses à l'état de poudre, on effectue la compatibilisation directement. On met les particules en suspension dans un milieu hydroalcoolique pour obtenir un sol minéral (a).

Dans le milieu hydroalcoolique de la suspension (a), un alcoxysilane de formule (1) s'hydrolyse en silanol susceptible de se condenser sur une ou plusieurs fonctions hydroxyle présentes à la surface de l'oxyde métallique.

La réaction globale de compatibilisation (greffage) peut donc être décrite de façon simple comme l'enchaînement des deux réactions suivantes :

1-hydrolyse de l'alcoxysilane

$$\equiv SiOR + H_2O ----> \equiv SiOH + ROH$$

2-condensation du silanol obtenue sur la surface de l'oxyde

$$\equiv MOH + \equiv SiOH ----> \equiv M\text{-}O\text{-}Si\equiv + H_2O$$

De nombreux composés de silicium sont envisageables pour effectuer un tel greffage par hydrolyse/condensation mais tous ne sont pas utilisables dans les conditions de réaction du procédé selon l'invention.

Les alcoxysilanes de formule (1) définis ci-dessus sont bien adaptés à la mise en oeuvre du procédé de l'invention, car ils sont solubles dans le milieu de greffage et ne s'hydrolysent pas trop vite dans ce milieu.

Un composé du silicium qui présenterait une grande aptitude à l'hydrolyse serait susceptible de se condenser sur lui-même et non pas sur les fonctions hydroxyle de surface du minéral. C'est le cas par exemple d'un disilazane.

Cet inconvénient est évité par le choix des alcoxysilanes de formule (1) dont la réactivité est modérée.

Parmi les composés de formule (1), il est préférable que le radical R représente un groupe méthyle ou éthyle.

De même, il est avantageux que R' représente un groupe octyle, dodécyle ou octadécyle.

En outre, on préfère les trialcoxysilanes, c'est-à-dire les composés tels que x = 3.

A titre d'exemple, on peut mentionner l'octyltriméthoxysilane (OTMS), le dodécyltriméthoxysilane (DTMS) ou le n-octadécyltriméthoxysilane (ODTMS).

Parmi les oxydes métalliques, on peut citer en particulier l'oxyde de titane, utilisé sous forme rutile ou anatase. Il peut s'agir notamment d'anatase lenticulaire ou plaquettaire.

Selon le procédé de l'invention, on prépare une suspension hydroalcoolique (a) de particules d'oxyde métallique non-nanoporeuses. Les particules d'oxyde se dispersent facilement dans le milieu.

Le rapport pondéral de l'eau à l'alcool dans cette suspension est avantageusement compris entre 0,2 et 0,8.

On opère de préférence avec une suspension diluée et la concentration en poids des particules dans cette suspension est de préférence comprise entre 5 g/l et 200 g/l.

La suspension hydroalcoolique (a) peut être acide ou basique et son pH varie selon l'oxyde à traiter.

De manière générale, lorsqu'on opère en milieu basique, le pH est avantageusement compris entre 9 et 12. La base utilisée pour fixer ce pH peut être choisie parmi les hydroxydes de métaux alcalins et notamment ceux de sodium, lithium et potassium, les carbonates de métaux alcalins et notamment ceux de sodium et de potassium, l'ammoniaque et les amines primaires, secondaires ou tertiaires, telles que la monoéthylamine ou la diéthylamine.

En particulier, il est préférable que le pH soit supérieur à PIE+2, où PIE est le point isoélectrique de l'oxyde à traiter.

De manière générale, lorsqu'on opère en milieu acide, le pH est avantageusement compris entre 1 et 5. L'acide utilisé pour fixer le pH peut être choisi parmi l'acide nitrique, l'acide chlorhydrique, l'acide acétique et d'autres acides organiques.

En particulier, il est préférable que le pH soit inférieur à PIE-2, où PIE est le point isoélectrique de l'oxyde à traiter.

On ajoute ensuite une solution alcoolique anhydre (b) comprenant au moins un alcoxysilane de formule (1) avec un faible débit en prenant soin d'entretenir la dispersion des matières dans le milieu réactionnel. Cela permet d'éviter les risques résiduels d'auto-condensation du ou des alcoxysilanes.

De préférence, l'alcool de la solution (b) est identique à celui de la suspension hydroalcoolique (a).

L'apport d'alcoxysilane varie en fonction de la taille des particules d'oxyde à traiter et le rapport pondéral

3

de la quantité totale d'alcoxysilane (1) à la quantité d'oxyde peut être de 1 à 60%.

La température du milieu réactionnel lors de cet ajout se situe avantageusement entre 15 et 70°C, de préférence à la température ambiante.

Au cours de l'ajout, il se produit une floculation dans la suspension indiquant un changement des propriétés interfaciales des particules d'oxyde.

Après la fin de l'ajout, il est préférable de laisser la réaction se poursuivre pendant une à deux heures.

Après l'achèvement de réaction, les particules compatibilisées peuvent être isolées soit sous forme de poudre, soit sous forme de suspension organique par agitation avec un liquide organique.

Pour obtenir une poudre, le mélange réactionnel est soumis à une séparation liquide/solide, avantageusement par centrifugation. Le solide séparé est lavé à l'alcool puis séché à basse température, par exemple environ 60°C.

Lorsque le greffage de l'alcoxysilane (1) s'est déroulé en milieu ammoniacal, le mélange réactionnel est de préférence soumis à une étape de distillation à basse température pour éliminer l'ammoniaque et l'alcool, préalablement à la séparation solide/liquide.

La poudre obtenue peut ensuite être mise en suspension dans un milieu organique.

Il a par ailleurs été constaté que si l'on agite le mélange réactionnel avec un liquide organique choisi parmi une huile naturelle ou de synthèse, un solvant hydrocarboné ou un solvant quelconque non miscible à l'eau, les particules compatibilisées migrent spontanément vers le milieu organique, en raison de leur caractère organophile.

En exploitant cette propriété, une variante du procédé de préparation selon l'invention a été mise au point, qui consiste à faire réagir la suspension hydroalcoolique (a) de particules d'oxyde métallique avec une solution alcoolique (b) comprenant au moins un alcoxysilane (1) en présence d'un liquide organique non miscible à l'eau, de sorte que la réaction a lieu dans un milieu biphasique. Ainsi, au fur et à mesure de l'avancement de la réaction de compatibilisation, les particules qui ont atteint le degré d'hydrophobie suffisant passent spontanément dans la phase organique dans laquelle elles sont aisément dispersibles. Les particules peuvent être isolées et séchées, mais cette mise en oeuvre permet surtout d'obtenir directement de bonnes dispersions en milieu organique.

On peut ainsi obtenir aisément une suspension organique de particules d'oxyde compatibilisées en s'affranchissant des étapes de séparation, lavage et séchage, si l'on opère en présence d'un liquide organique tout au long de la réaction ou bien simplement à la fin de la réaction de compatibilisation.

Dans le cas d'une application cosmétique, le liquide organique préféré pour réaliser cette opération est une huile et en particulier le palmitate d'isopropyle.

Les particules organophiles préparées selon le procédé de l'invention ont une excellente affinité pour les milieux organiques et une bonne dispersibilité dans ces milieux.

Avantageusement, on parfait l'état de dispersion d'une suspension organique contenant lesdites particules compatibilisées en ajoutant à la suspension un agent de stabilisation stérique tel qu'un tensioactif non ionique. Ce tensioactif a pour effet de stabiliser la suspension hautement dispersée.

Parmi ces tensioactifs, on peut citer notamment les alkylphénols éthoxylés et en particulier le nonylphénol éthoxylé.

On pourra également procéder à une redispersion mécanique aux ultrasons des suspensions pour maintenir un état de dispersion optimal des particules compatibilisées.

Un sol organique contenant les particules compatibilisées selon l'invention a un état de dispersion proche de celui de sol minéral hydroalcoolique de départ.

Cet état de dispersion peut être estimé en mettant à profit les propriétés d'absorption de la lumière ultraviolette par les suspensions de particules d'oxyde considérées.

On disperse les particules compatibilisées dans l'hexane, en ajoutant un agent stabilisant afin d'obtenir un sol stable, puis on mesure sur cette dispersion le rapport entre l'absorbance dans l'ultraviolet à 308 nm et l'absorbance dans le visible à 525 nm : $A_{308}/A_{525}$. Plus ce rapport est élevé, plus la suspension sera transparente dans le visible et absorbante dans l'UV, l'absorbance dans l'UV étant liée à la présence des particules d'oxyde et la transparence dans le visible, à l'état de dispersion des particules dans la suspension.

On compare enfin ce rapport à celui obtenu pour une dispersion dans l'eau de particules de départ, non compatibilisées et donc hydrophiles, dont l'état de dispersion représente le maximum pouvant être atteint. Un traitement de compatibilisation sera jugé d'autant plus médiocre que la valeur du rapport $A_{308}/A_{525}$ mesuré sur la dispersion organique sera faible par rapport à celle du sol aqueux de particules de départ. Ce traitement sera jugé optimal lorsque la valeur du rapport $A_{308}/A_{525}$ mesuré sur la dispersion organique sera supérieure ou égale à celle correspondant au sol aqueux de particules de départ.

On constate que les dispersions organiques de particules organophiles préparées par un procédé selon l'invention ont un état de dispersion comparable, voire égal à l'état de dispersion du sol aqueux de particules hydrophiles de départ. Les suspensions organiques peuvent donc être qualifiées de "hautement dispersées".

La haute dispersibilité des particules organophiles préparées selon l'invention les rend particulièrement adaptées à toutes les applications des oxydes métalliques en milieu organique, par exemple comme charge de renforcement de matériaux.

On peut avantageusement mettre à profit les propriétés d'absorption des rayons ultraviolets par les dispersions organiques de particules d'oxyde organophiles obtenues par un procédé décrit précédemment.

Ainsi, l'invention concerne également l'utilisation de ces particules d'oxyde métallique organophiles en tant qu'agent anti-UV dans des plastiques tels que le polyéthylène ou le polypropylène. La bonne dispersion des particules au sein de la matrice polymère permet de conserver les propriétés optiques du polymère non chargé. En outre, l'effet anti-UV des présentes particules d'oxyde est comparable à celui obtenu avec des anti-UV organiques classiques.

En particulier, les nanoparticules d'oxyde de titane compatibilisées possèdent des propriétés de permanence dans les plastiques, contrairement aux anti-UV organiques monomères qui, dans le temps, migrent en surface du film et perdent ainsi leur activité. Les anti-UV organiques polymères, eux, migrent peu ou pas, mais cela se fait au détriment de leur performance anti-UV. Les nanoparticules de $TiO_2$ compatibilisées selon l'invention optimisent ainsi l'équilibre efficacité anti-UV/permanence dans le plastique.

En outre, les particules d'oxydes de cérium ou de titane rendues organophiles peuvent également être utilisées en tant qu'agent anti-UV dans des produits cosmétiques organiques liquides ou solides. Les poudres d'oxyde ainsi que les dispersions organiques de ces particules organophiles ont en effet une très faible absorption dans le visible et une forte absorption dans l'ultraviolet. Incorporées dans une formulation cosmétique, notamment sous forme de crème ou de poudre, elles permettent d'obtenir des produits qui, une fois étalés sur la peau, sont transparents et pourtant fortement protecteurs contre les rayons ultraviolets.

Il est également à noter que les particules d'oxyde organophiles sont parfaitement hydrophobes et chimiquement inertes.

En effet, les oxydes métalliques, en particulier de titane, non traités ont une forte tendance à dégrader les molécules organiques environnantes.

Dans le cas présent, on suppose que la greffe de l'alcoxysilane (1) forme un enrobage qui protège la surface de l'oxyde minéral.

Les particules obtenues selon l'invention représentent donc des charges ou additifs particulièrement intéressants qui ne favorisent pas la dégradation du produit final dans lequel elles sont incorporées.

L'invention a donc également pour objet les particules organophiles et les suspensions de particules organophiles obtenues par un procédé décrit précédemment.

La présente invention est illustrée par les exemples suivants :

## EXEMPLES

## EXEMPLE 1

Dans cet exemple, les particules à traiter sont des particules d'oxyde de titane sous forme d'anatase plaquettaire obtenu par synthèse hydrothermale et autoclavage. Ces particules monocristallines ont une dimension approchant la dizaine de nanomètres et sont fortement agrégées (taille des agrégats ≈ 100 à 200 nm). La surface des particules ne comporte pas de pores de taille inférieure à 5 nm.

Dans un réacteur agité à 500 tours par minute, on prépare un pied de cuve en mélangeant, à 25°C, 400 ml d'eau permutée, 600 ml d'éthanol pur et 10 g d'oxyde de titane. Le pH de la suspension hydroalcoolique est ajusté à 2 en ajoutant la quantité nécessaire d'acide nitrique.

Une solution contenant 1,6 g d'octyltriméthoxysilane dans 50 ml d'éthanol est ajoutée à un débit de 15 ml/heure au pied de cuve. En fin d'ajout, on obtient une suspension floculée qui sédimente dans le réacteur.

100 ml de palmitate d'isopropyle sont versés dans la suspension floculée et, après agitation, on constate que le solide passe dans la phase organique. La suspension huileuse est séparée et on y ajoute 10% de nonylphénol éthoxylé 9OE (CEMULSOL NP9) pour la stabiliser.

La suspension reste stable pendant plusieurs semaines.

Pour caractériser la dispersibilité des particules compatibilisées, on effectue la même réaction mais on sépare le solide sédimenté après l'ajout d'OTMS. Ce solide est lavé avec des alcools puis séché à 60°C.

On prépare une suspension de ce solide dans l'hexane en présence de nonylphénol éthoxylé, puis on mesure l'absorbance de cette suspension dans l'ultraviolet à 308 nm ($A_{308}$) et dans le visible à 525 nm ($A_{525}$).

Le rapport $A_{308}/A_{525}$, indicatif de l'état de dispersion de la suspension, est comparé au même rapport correspondant à une suspension aqueuse des particules d'oxyde non compatibilisées, qui sert de référence comme suspension parfaitement dispersée.

Pour la suspension organique de particules compatibilisées, $(A_{308}/A_{525})_{org}$ vaut 2,4.

Pour le sol aqueux de particules de départ non compatibilisées, $(A_{308}/A_{525})_{aq}$ vaut 2,5.

Le rapport $(A_{308}/A_{525})_{org}$ est très légèrement inférieur au rapport de la référence aqueuse, ce qui indique que la dispersibilité en milieu organique des particules compatibilisées est excellente.

Les particules solides préparées selon ce mode opératoire sont soumises à un test d'hydrophobie sur produit sec. Après avoir déposé une goutte d'eau sur la poudre, on observe que la goutte ne s'étale pas et reste sous forme de goutte sphérique.

On évalue également le degré de protection de la surface de l'oxyde grâce à un test colorimétrique. On estime ainsi l'aptitude de la particule compatibilisée à dégrader un milieu organique.

On malaxe un peu de poudre avec quelques gouttes d'un mélange $H_2SO_4$-$H_2O_2$. On développe alors plus ou moins rapidement une couleur allant du jaune pâle au rouge sang et correspondant au complexe $TiO_2^{2+}$. On classe la qualité de l'enrobage à partir de la couleur développée au bout d'un temps bien défini.

| couleur développée | orange-rouge | jaune orangé | jaune doré | jaune pâle |
|---|---|---|---|---|
| qualification du degré de protection | 0 mauvaise protection | + | ++ | +++ très bonne protection |

D'après ce test, les particules de l'exemple 1 ont une bonne protection de la surface (++).

## EXEMPLE 2

Dans cet exemple, les particules à traiter sont des particules d'oxyde de titane sous forme anatase lenticulaire (obtenu par thermohydrolyse d'oxychlorure de titane en présence d'acide citrique). Ces particules polycristallines (taille de cristallite $\approx$ 6 nm) ont un diamètre de 30 à 50 nm, et sont bien individualisées. Des mesures de porosité ont montré que ces particules lenticulaires d'anatase possèdent des pores de taille inférieure à 5 nm.

Dans un premier temps, on réalise donc un prétraitement consistant à éliminer cette nanoporosité.

Dans un réacteur agité à 500 tours/minute, on prépare un pied de cuve en mélangeant, à 25°C, 240 ml d'eau permutée, 500 ml d'éthanol pur, 260 ml d'une solution aqueuse d'ammoniaque à 25% en $NH_3$ et 10 g d'oxyde de titane.

On effectue le prétraitement de surface en ajoutant à un débit de 15 ml/h une solution contenant 12 g de tétraéthylorthosilicate dans 50 ml d'éthanol. En fin d'ajout, on obtient une suspension stable.

On procède ensuite à la compatibilisation en ajoutant à un débit de 15 ml/h une solution contenant 5 g d'octyltriméthoxysilane dans 10 ml d'éthanol. En fin de réaction, on obtient une suspension floculée qui sédimente dans le réacteur.

100 ml de palmitate d'isopropyle sont versés dans la suspension floculée et, après agitation, le solide passe dans la phase organique. La suspension huileuse est séparée et on y ajoute 10% de nonylphénoléthoxylé (CEMULSOL NP9).

La suspension reste stable pendant plusieurs semaines.

On caractérise la dispersibilité des particules compatibilisées comme à l'exemple 1.

Pour la suspension organique de particules compatibilisées, $(A_{308}/A_{525})_{org}$ vaut 9.

Pour le sol aqueux de particules de départ non compatibilisées, $(A_{308}/A_{525})_{aq}$ vaut 8,5.

La dispersibilité des particules compatibilisées est excellente.

Les particules compatibilisées de l'exemple 2 manifestent une hydrophobie parfaite et une très bonne protection de la surface (+++) d'après le test colorimètrique.

## EXEMPLE COMPARATIF

A titre de comparaison, les particules d'anatase lenticulaire nanoporeuses traitées dans l'exemple 2 sont soumises directement à la compatibilisation à l'octyltriméthoxysilane, sans procéder au prétraitement de surface destiné à éliminer la nanoporosité.

Dans un réacteur agité à 500 tours/minute, on prépare un pied de cuve en mélangeant, à 25°C, 240 ml d'eau permutée, 500 ml d'éthanol pur, 260 ml d'une solution aqueuse d'ammoniaque à 25 % en $NH_3$ et 10 g desdites particules d'oxyde de titane nanoporeuses.

On ajoute à ce pied de cuve une solution contenant 5 g d'octyltriméthoxysilane dans 10 ml d'éthanol, à un débit de 15 ml/h. En fin de réaction, on obtient une suspension floculée qui sédimente dans le réacteur.

Comme à l'exemple 2, on verse du palmitate d'isopropyle dans la suspension floculée, mais même après une agitation très vigoureuse, il n'est pas possible de faire passer le solide dans la phase organique.

Le solide est séparé du milieu réactionnel par décantation, lavé à l'éthanol et séché à 60°C.

On apprécie la dispersibilité des particules en les mettant en suspension dans l'hexane en présence de nonylphénol éthoxylé (CEMULSOL NP9), comme dans l'exemple 1. Pour cette suspension organique de particules compatibilisées non prétraitées, le rapport $(A_{308}/A_{525})_{org}$ vaut 3,9 alors que, pour le sol aqueux de particules de départ nanoporeuses non-compatibilisées, le rapport $(A_{308}/A_{525})_{aq}$ vaut 8,5 et, pour le sol organique de particules prétraitées et compatibilisées de l'exemple 2, le rapport $(A_{308}/A_{525})_{org}$ vaut 9.

Le rapport $(A_{308/525})_{org}$ des particules compatibilisées non-prétraitées est largement inférieur au rapport de la référence aqueuse, ce qui indique que la dispersibilité en milieu organique des particules compatibilisées, mais encore nanoporeuses, est très mauvaise.

A titre indicatif, le rapport $(A_{308}/A_{525})_{org}$ pour une suspension dans l'hexane, en présence de CEMULSOL NP9, des particules minérales de départ nanoporeuses non compatibilisées vaut 2,5. L'amélioration de la dispersibilité apportée par la compatibilisation est très faible.

Les particules compatibilisées ainsi isolées manifestent une hydrophobie moyenne (leur extraction par une phase organique est impossible) mais une bonne protection de la surface (+++) d'après le test colorimétrique.

## EXEMPLE 3

Dans cet exemple, les particules à traiter sont des particules d'oxyde de titane sous forme anatase lenticulaire (obtenu par thermohydrolyse d'oxychlorure de titane en présence d'acide citrique). Ces particules polycristallines (taille de cristallite = 6 nm) ont un diamètre de 20 à 30 nm, et sont donc bien individualisées. Des mesures de porosité ont montré que ces particules lenticulaires d'anatase possèdent des pores de taille inférieure à 5 nm.

Dans un premier temps, on réalise donc un prétraitement consistant à éliminer cette nanoporosité.

Dans un réacteur agité à 500 tours/minute, on prépare un pied de cuve en mélangeant, à 25°C, 240 ml d'eau permutée, 500 ml d'éthanol pur, 260 ml d'une solution aqueuse d'ammoniaque à 25% en $NH_3$ et 10 g d'oxyde de titane.

On effectue le prétraitement de surface en ajoutant à un débit de 15 ml/h une solution contenant 12 g de tétraéthylorthosilicate dans 50 ml d'éthanol. En fin d'ajout, on obtient une suspension stable.

On procède ensuite à la compatibilisation en ajoutant à un débit de 15 ml/h une solution contenant 5 g d'octyltriméthoxysilane dans 10 ml d'éthanol. En fin de réaction, on obtient une suspension floculée qui sédimente dans le réacteur.

On laisse mûrir cette suspension pendant 2 heures sous agitation.

Puis on procède à une distillation sous vide pour éliminer l'éthanol et l'ammoniaque à une température de 70-80°C sous une pression de 8 kPa (environ 60 mm de Hg). Lorsqu'environ 200 ml de distillat sont recueillis, on laisse refroidir le réacteur, on rétablit la pression normale et on ajoute de l'eau dans le réacteur de manière à réajuster au volume initial. On renouvelle alors ces opérations pour éliminer l'éthanol et l'ammoniaque résiduels.

Lorsque le volume dans le réacteur n'est plus que de 500-600 ml, le mélange commence à mousser, la dispersion paraît savonneuse et nacrée. On laisse alors refroidir. Le contenu du réacteur est récupéré.

La charge totale du réacteur est séparée en deux fractions égales qui sont centrifugées. On laisse décanter la couche aqueuse supérieure. Le gâteau de centrifugation est remis en suspension dans 100 ml d'éthanol absolu, bien mélangé puis centrifugé.

On décante de nouveau la couche supérieure et le gâteau de centrifugation est remis en suspension dans 100 ml d'éthanol absolu, bien mélangé puis centrifugé. Le gâteau obtenu est assez compact, ferme. On le laisse sécher à l'air pour obtenir une poudre.

On caractérise la dispersibilité des particules compatibilisées comme dans l'exemple 1. Pour la suspension organique de particules compatibilisées, le rapport $(A_{308}/A_{525})_{org}$ vaut 26. Pour le sol aqueux de particules de départ non compatibilisées, le rapport $(A_{308}/A_{525})_{aq}$ vaut 30. Le rapport $(A_{308}/A_{525})_{org}$ est proche de $(A_{308}/A_{525})_{aq}$' ce qui indique que la dispersibilité en milieu organique des particules compatibilisées est excellente.

## EXEMPLE 4

Cet exemple illustre un procédé de compatibilisation en milieu biphasique. Les particules à traiter sont les mêmes que dans l'exemple 3, et on les soumet de façon préliminaire à un prétraitement visant à éliminer la nanoporosité.

Dans un réacteur agité à 500 tours/minute, on prépare un pied de cuve en mélangeant, à 25°C, 240 ml d'eau permutée, 500 ml d'éthanol pur, 260 ml d'une solution aqueuse d'ammoniaque à 25% en $NH_3$ et 10 g d'oxyde de titane.

On effectue le prétraitement de surface en ajoutant à un débit de 15 ml/h une solution contenant 12 g de tétraéthylorthosilicate dans 50 ml d'éthanol. En fin d'ajout, on obtient une suspension stable.

On procède ensuite à la compatibilisation en présence d'une phase organique non miscible surnageante.

On ajoute 80 ml de palmitate d'isopropyle sur la suspension précédemment obtenue. On ajoute à un débit de 15 ml/h une solution contenant 4 g d'octyltriméthoxysilane dans 10 ml d'éthanol, l'addition se faisant sous agitation à 500 tours/minute.

En fin d'addition, le contenu du réacteur est chauffé à 60°C pendant 7 heures. La couche organique devient alors laiteuse. On récupère cette phase constituée de particules de $TiO_2$ compatibilisées en suspension dans le palmitate d'isopropyle.

## EXEMPLE 5

On effectue la compatibilisation de particules d'oxyde de cérium.

Dans un réacteur agité à 500 tours/minute, on prépare un pied de cuve en mélangeant, à 25°C, 500 ml d'eau permutée, 500 ml d'éthanol pur et 5g de colloïde d'hydrate de cérium. Le pH de la suspension hydroalcoolique est ajusté à 3,5 avec la quantité nécessaire d'acétate de sodium.

Une solution contenant 1 g d'octyltriméthoxysilane dans 50 ml d'éthanol est ajoutée au pied de cuve à un débit de 15 ml/h. On obtient une suspension floculée qui sédimente dans le réacteur.

100 ml de palmitate d'isopropyle sont versés dans la suspension floculée et, après agitation, le solide passe en phase organique.

On a pu également vérifier que la poudre formée des particules compatibilisées de cet exemple est hydrophobe.

## EXEMPLE 6

Dans cet exemple, les particules à traiter sont des particules de silice non nanoporeuses.

Pour effectuer la compatibilisation, on prépare 279 g d'une suspension de silice dans l'eau contenant 50 g de silice (soit 21,5% en poids) homogénéisée aux ultrasons. L'opération étant exothermique, la température de la suspension s'élève à 60°C environ et on la laisse refroidir à l'ambiante avant de la couler dans un réacteur équipé d'un système d'agitation et d'une ampoule d'introduction. On prépare un pied de cuve en y ajoutant 306,7 g d'une solution d'ammoniaque à 20% et 542 ml d'éthanol absolu, sous une agitation de 500 tours/minute.

On introduit ensuite par l'ampoule de coulée au goutte à goutte 31,6 g de triméthoxyoctylsilane en solution dans 92 ml d'éthanol absolu, tout en agitant le mélange. Après la fin de l'ajout, on fait mûrir le mélange de sorte que les particules soient mises en contact avec le triméthoxyoctylsilane pendant au total 5 heures.

Après le mûrissement, une part du mélange est prélevée et mise en contact avec du palmitate d'isopropyle et l'on observe la migration immédiate des particules vers la phase organique.

Le reste du mélange réactionnel est centrifugé et décanté, le gâteau de centrifugation est repris avec de l'éthanol à 95%. Cette opération est effectuée 3 fois, comme dans l'exemple 3.

Le dernier gâteau obtenu est séché à l'air pour obtenir 75 g de poudre.

Les particules ainsi compatibilisées manifestent une hydrophobie parfaite.

## EXEMPLE 7

On utilise les particules d'oxyde de titane compatibilisées de l'exemple 2 comme agent anti-UV dans un polymère de polypropylène.

On incorpore 0,1 à 0,2% en poids de particules de $TiO_2$ dans du polypropylène.

On forme un film de ce polymère ayant une épaisseur de 200 µm. Ce film conserve la transparence du polymère non chargé.

L'action anti-UV de ces particules est satisfaisante et comparable à celle d'agents anti-UV organiques, introduits à raison de 0,1% en poids du polymère.

Pour comparaison, on incorpore 0,1% en poids d'oxyde de titane du type "pigment pour peinture" (particules de 0,2 µm à 0,3 µm de diamètre). Un film de 200 µm d'épaisseur obtenu à partir de ce polymère est blanc. En outre, ces particules d'oxyde de titane accélèrent la dégradation du film.

## Revendications

1. Procédé de préparation de particules organophiles d'un oxyde métallique choisi parmi l'alumine $Al_2O_3$, la silice $SiO_2$, les oxydes de métaux de transition, notamment l'oxyde de titane $TiO_2$, et les oxydes de terres rares, notamment l'oxyde de cérium $CeO_2$, par lequel on fait réagir une suspension hydroalcoolique (a) de particules d'oxyde métallique dont la surface ne possède pas de pores de diamètre inférieur à 5 nm, avec une solution alcoolique anhydre (b) comprenant au moins un alcoxysilane de formule (1)

$$Si(OR)_x R'_{4-x} \qquad (1)$$

dans laquelle R représente un groupe alkyle comportant de 1 à 6 atomes de carbone, R' représente un groupe hydrocarboné choisi parmi les groupes alkyle, cycloalkyle, aryle, alkylaryle, arylalkyle, alcényle et alcynyle comportant au moins 1 atome de carbone et x est un entier de 1 à 3, lesdits alcools, identiques ou différents, comportant de 1 à 5 atomes de carbone.

2. Procédé selon la revendication 1, dans lequel les particules d'oxyde métallique à traiter ont un diamètre moyen inférieur ou égal à 100 nm.

3. Procédé selon la revendication 1 ou 2 dans lequel, dans une étape préliminaire, on traite des particules d'oxyde métallique nanoporeuses de façon à ce que leur surface ne comporte pas de pores de diamètre inférieur à 5 nm.

4. Procédé selon la revendication 3 dans lequel, dans ladite étape préliminaire, on fait réagir une suspension hydroalcoolique (c) de particules nanoporeuses d'oxyde métallique avec au moins un tétraalcoxysilane de formule (2) $Si(OR'')_4$, dans laquelle R'' représente un groupe alkyle comportant de 1 à 5 atomes de carbone, pour obtenir une suspension hydroalcoolique (a) de particules d'oxyde dont la surface ne comporte pas de pores de diamètre inférieur à 5 nm.

5. Procédé selon la revendication 3 dans lequel, dans ladite étape préliminaire, les particules d'oxyde nanoporeuses sont soumises à un traitement thermique en autoclave.

6. Procédé selon l'une des revendications 1 à 5, dans lequel R est le groupe méthyle ou éthyle.

7. Procédé selon l'une des revendications 1 à 6, dans lequel R' est le groupe octyle, dodécyle ou octadécyle.

8. Procédé selon l'une des revendications 1 à 7, dans lequel x vaut 3.

9. Procédé selon l'une des revendications 1 à 8, dans lequel l'oxyde métallique est l'oxyde de titane.

10. Procédé selon l'une des revendications 1 à 9, dans lequel le rapport pondéral de la quantité totale d'alcoxysilane de formule (1) à la quantité d'oxyde à traiter est de 1 à 60%.

11. Procédé selon l'une des revendications 1 à 10, dans lequel le rapport pondéral de l'eau à l'alcool dans la suspension hydroalcoolique (a) est de 0,2 à 0,8.

12. Procédé selon l'une des revendications 1 à 11, dans lequel l'alcool de la suspension hydroalcoolique (a) et l'alcool de solution alcoolique anhydre (b) sont identiques.

13. Procédé selon l'une des revendications 1 à 12, dans lequel le pH de la suspension hydroalcoolique (a) est de 1 à 5.

14. Procédé selon l'une des revendications 1 à 12, dans lequel le pH de la suspension hydroalcoolique (a) est de 9 à 12.

15. Procédé selon l'une des revendications 1 à 14, dans lequel la concentration en particules d'oxyde métallique non-nanoporeuses dans la suspension hydroalcoolique (a) est de 5 g/l à 200 g/l.

16. Procédé selon l'une des revendications 1 à 15, dans lequel la réaction entre (a) et (b) est effectuée à une température de 15 à 70°C.

17. Procédé selon l'une des revendications 1 à 16, dans lequel la réaction entre (a) et (b) se fait à température ambiante.

**18.** Procédé selon l'une des revendications 1 à 17, dans lequel le mélange issu de la réaction entre (a) et (b) est soumis à une séparation liquide/solide, le solide résultant est lavé avec un alcool puis séché.

**19.** Procédé selon l'une des revendications 1 à 18, dans lequel la réaction entre (a) et (b) est effectuée en présence d'un liquide organique non miscible à l'eau dans lequel les particules migrent au fur et à mesure de leur compatibilisation.

**20.** Procédé de préparation d'une suspension organique de particules d'oxyde métallique organophiles dans lequel on met en oeuvre un procédé selon la revendication 18, on met les particules solides issues du séchage en suspension dans un solvant organique choisi parmi une huile naturelle ou de synthèse, un solvant hydrocarboné ou un solvant quelconque non miscible à l'eau, et on ajoute à la suspension organique ainsi obtenue un agent de stabilisation choisi parmi les tensioactifs non ioniques.

**21.** Procédé de préparation d'une suspension organique de particules d'oxyde métallique organophiles dans lequel on met en oeuvre un procédé selon l'une quelconque des revendications 1 à 17, on agite le mélange issu de la réaction entre (a) et (b) avec un solvant organique choisi parmi une huile naturelle ou de synthèse, un solvant hydrocarboné ou un solvant quelconque non miscible à l'eau, ce par quoi les particules d'oxyde métallique organophiles passent en suspension dans le liquide organique, et on ajoute à la suspension organique ainsi obtenue un agent de stabilisation choisi parmi les tensioactifs non-ioniques.

**22.** Procédé selon l'une quelconque des revendications 19 à 21, dans lequel le solvant est le palmitate d'isopropyle ou le stéarate d'isopropyle.

**23.** Utilisation de particules d'oxyde métallique organophiles obtenues par un procédé selon l'une des revendications 1 à 19 en tant qu'agent anti-UV dans des plastiques.

**24.** Utilisation de particules d'oxyde de cérium ou de titane organophiles obtenues par un procédé selon l'une des revendications 1 à 19 en tant qu'agent anti-UV dans des produits cosmétiques organiques liquides ou solides.

**25.** Particules organophiles d'oxyde métallique obtenues par un procédé selon l'une quelconque des revendications 1 à 19.

**26.** Suspension de particules organophiles d'un oxyde métallique, obtenue par un procédé selon l'une quelconque des revendications 19 à 22.

Office européen
des brevets

# RAPPORT DE RECHERCHE EUROPEENNE

Numero de la demande

EP 95 40 1442

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | EP-A-0 216 047 (THE SHERWIN-WILLIAMS COMPANY)<br><br>* page 3, ligne 26 - page 4, ligne 4 *<br>* page 5, ligne 6 - page 6, ligne 16 *<br>* page 7, ligne 1 - page 8, ligne 3; revendications 1,6,7,9 *<br>--- | 1,6-8, 10,11, 13,15, 16,25 | C09C3/12<br>C09C1/36<br>C09C1/30<br>C08K9/06<br>A61K7/42<br>C09D17/00 |
| X | US-A-5 013 585 (TAKAAKI SHIMIZU ET AL.)<br><br>* colonne 2, ligne 13 - ligne 45; revendications 1,5 *<br>--- | 1,4,6,7, 25 | |
| A | GB-A-1 376 706 (KANEGAFUCHI KAGAKU KOGYO K. K.)<br>* page 2, ligne 89 - ligne 116 *<br>--- | 1,6,10, 23,25 | |
| A | GB-A-1 154 835 (MIDLAND SILICONES)<br><br>* page 1, ligne 70 - page 2, ligne 6; revendications 1,5,7 *<br>--- | 1,4,6,9, 23 | |
| A | FR-A-2 116 018 (DEGUSSA)<br>* revendication 1 *<br>--- | 4 | **DOMAINES TECHNIQUES RECHERCHES (Int.Cl.6)**<br><br>C09C<br>C09D |
| A | CHEMICAL ABSTRACTS, vol. 94, no. 6, 9 Février 1981 Columbus, Ohio, US; abstract no. 32299m, page 90; colonne L; * abrégé * & JP-A-80 110 163 (MALVERN MINERALS) 25 Août 1980<br>--- | 1,9,25 | |
| A | EP-A-0 523 654 (CANON)<br>* revendications 2-6 *<br>----- | 1,9,25 | |

Le présent rapport a été établi pour toutes les revendications

| Lieu de la recherche | Date d'achèvement de la recherche | Examinateur |
|---|---|---|
| LA HAYE | 1 Août 1995 | Van Bellingen, I |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

EPO FORM 1503 03.82 (P04C02)